# EUROPEAN PATENT SPECIFICATION

(11) **EP 3 761 315 B1**
(45) Date of publication and mention of the grant of the patent: **18.12.2024**
(21) Application number: 19425048.6
(22) Date of filing: 03.07.2019
(51) Int. Cl.: G16H 20/13, G16H 40/20

(54) **LABELLING METHOD AND SYSTEM FOR TAGGING DRUGS TO BE ADMINISTERED TO A PATIENT IN A HOSPITAL OR SURGICAL SETTING**
ETIKETTIERVERFAHREN UND SYSTEM ZUR MARKIERUNG VON AN EINEN PATIENTEN ZU VERABREICHENDEN ARZNEIMITTELN IN EINEM KRANKENHAUS ODER EINER CHIRURGISCHEN UMGEBUNG
SYSTÈME ET PROCÉDÉ D'ÉTIQUETAGE POUR ÉTIQUETER DES MÉDICAMENTS DEVANT ÊTRE ADMINISTRÉS À UN PATIENT EN MILIEU HOSPITALIER OU CHIRURGICAL

(43) Date of publication of application: 06.01.2021
(60) Divisional application: 24165954.9
(73) Proprietor: Webbit S.r.l., 20090 Trezzano sul Naviglio (MI) (IT)
(72) Inventor: CAIS, Fulvio, I-20088 Gudo Visconti (MI) (IT)
(74) Representative: Botti & Ferrari S.p.A.

(56) References cited:
- US-A1- 2007 250 346
- US-A1- 2008 097 787
- ANONYMOUS: "Fact sheet. Pharmacy labelling. Zebra's Pharmacy Labelling Solutions. Identify medicines safely and easily.", 31 January 2018 (2018-01-31), XP055650173, Retrieved from the Internet <URL:https://www.zebra.com/content/dam/zebra_new_ia/en-us/solutions-verticals/vertical-solutions/healthcare/fact-sheet/healthcare-pharmacy-labelling-fact-sheet-gb-en.pdf> [retrieved on 20191205]
- ANONYMOUS: "ZEBRA Thermal Printers. Demanding Work Demands the Best.", 31 December 2018 (2018-12-31), XP055650174, Retrieved from the Internet <URL:https://www.zebra.com/content/dam/zebra_new_ia/en-us/solutions-verticals/product/Printers/General/brochures/printers-brochure-portfolio-en-us.pdf> [retrieved on 20191205]
- ANONYMOUS: "PRODUCT SPEC SHEET. ZD620-HC PERFORMANCE HEALTHCARE DESKTOP PRINTERS. ZD620 Performance Healthcare Desktop Printers", 31 December 2018 (2018-12-31), XP055650179, Retrieved from the Internet <URL:https://www.zebra.com/content/dam/zebra_new_ia/en-us/solutions-verticals/product/Printers/Desktop%20Printers/zd620-series-desktop-printers/zd620-healthcare/specification-sheet/zd620hc-specification-sheet-a4.pdf> [retrieved on 20191205]
- ANONYMOUS: "Technical specifications. ZD620 Printers. ZD620TM Printer Specifications", 18 December 2017 (2017-12-18), XP055650180, Retrieved from the Internet <URL:https://www.zebra.com/content/dam/zebra_new_ia/en-us/solutions-verticals/product/Printers/Desktop%20Printers/zd620-series-desktop-printers/tech-specs/zd620-technical-specifications-en-us.pdf> [retrieved on 20191205]
- ANONYMOUS: "Zebra(TM) IQ Color", 31 December 2010 (2010-12-31), XP055650184, Retrieved from the Internet <URL:https://www.zebra.com/content/dam/zebra_new_ia/en-us/solutions-verticals/product/Supplies/Labels%20and%20Tags/GENERAL/zebra-iq-%20datasheet-gb.pdf> [retrieved on 20191205]
- ANONYMOUS: "MATERIALS SPEC SHEET. Z-Perform 1000D", 31 December 2018 (2018-12-31), XP055650290, Retrieved from the Internet <URL:https://www.zebra.com/content/dam/zebra_new_ia/en-us/solutions-verticals/product/Supplies/Laser,%20Thermal%20and%20RFID%20Wristbands/z-perform/spec-sheet/z-perform-1000d-spec-sheet-en-us.pdf> [retrieved on 20191206]
- ANONYMOUS: "CognitiveTPG. C Series Compact Industrial Thermal Label Printer.", 31 December 2014 (2014-12-31), XP055650305, Retrieved from the Internet <URL:https://cdn.barcodegiant.com/themes/barcodesinc/pdf/CognitiveTPG/c-series.pdf> [retrieved on 20191206]

## Description

### Field of application

The present invention relates to a labelling method for identifying drugs to be administered to a patient.

The invention also concerns a system implementing said method.

The invention can find a useful application in several medical settings, both for the supply of therapies in hospital recovery wards and for the intraoperative administration of drugs during surgery.

### Prior art

In the above-identified fields, the need to prepare a drug transferring it in containers of different nature intended for the final administration to the patient, such as for example syringes, syringe pumps, infusion bags, bottles, etc, is known.

In these cases, it is naturally imperative to avoid drug exchanges having potentially life-threatening consequences. For this reason, it is customary to put a label on the container being used, to identify the drug contained therein at the time of the actual administration.

Traditionally, the label is manually filled in by the operator that is working to transfer the drug. But this solution still involves a high risk of human error, mainly linked to the possible misunderstandings in interpreting the handwriting.

Moreover, the whole tracking system of the administered drugs is based on manual filling operations referred to the operator, with possible incidental, when not malicious, errors.

In this context, the use of pre-printed labels that facilitate the reading, but do not improve the tracking of drugs, nor remove the risk of human error in consideration of the possible confusion of the operator at the time of label selection, has been recently suggested.

A further contribution to the safety of hospital procedures came from the introduction of real-time label printing systems, that allow the operator to obtain the label required to identify the specific drug to be employed. But these printing systems, although ensuring great advantages in terms of safety and tracking, significantly slow down the procedures for preparing the drugs by the operators.

In fact, it must be considered that, in the hospital standard practice, it is normally required to prepare at the same time different drugs to be administered to the patient in the context of a therapy or a surgery protocol. The operator has thus to create and print a plurality of labels, to be individually put on different containers, repeatedly shifting the attention between the system terminal and the containers themselves, with non-negligible data entry times.

Moreover, the above systems have a residual risk of error when transferring the labels, in particular when an operator - to accelerate process times - decides to put the labels at a later time after printing operations. In this case, he has to distinguish among a plurality of printed labels, directed to different drugs to be administered to different patients, and the possibility of confusion is high.

For the above reasons, the real-time printing systems did not experience a widespread distribution, at least in European hospitals.

A method for processing a multiple prescription order and pre-packaging the multiple medications in a single container at the production facility, comprising the step of printing an instruction leaflet, is disclosed in prior art document US 2007/250346 A1.

US2008/0097787 A1 concerns pre-cut adhesive multi-dose pharmacy labels suitable for point-of-sale printing of information thereon. This document discloses a label comprising information about the different drugs to be administered to a specific patient and the label content comprising a printing field for each drug to be administered to the patient and each of said printing fields comprising at least one identifier of the respective drug.

The technical problem underlying the present invention is therefore to provide a method and a related labelling system for identifying drugs to be administered to a patient in a hospital or surgical setting which solve the above-reported drawbacks, and which allow in particular a safe and fast labelling.

### Summary of the invention

The scope of the invention is defined by the appended claims 1-12. The examples, aspects and embodiments, if any, disclosed in the following description that do not fall within the scope of the claims are for reference only and are to be interpreted as examples useful for understanding various embodiments of the invention.

The above-identified technical problem is solved by a labelling method for identifying drugs to be administered to a patient in a hospital or surgical setting, as defined by the appended claim 1.

The method of claim 1 advantageously allows the information about the whole therapy to be administered to the patient to be obtained on a single support; the operator is thus facilitated in identifying and preparing in sequence the different drugs, putting where appropriate the respective label on the respective container.

The risks of error and confusion are therefore considerably reduced, by suggesting a linear and rational labelling method that leads the operator avoiding process ambiguities.

The method, by grouping the selection and printing operations related to the different drugs of a single therapy together, considerably reduces process times, being also compatible with the working rates of the most frantic wards, such as emergency room ones.

Said support strip is unwound from an endless feeding strip, said method comprising a step of automatically size-cutting said endless feeding strip.

The above automatic cutting operation ensures that the strips related to different patients are separated from each other, avoiding any risk of confusion for the operator.

Preferably, the generated and printed printing content further comprises a header printing field, comprising the name and/or an ID of the patient. This header field clearly identifies the patient which the therapy described in the following drug list refers to, warding off replacements and increasing the process safety.

In said step of printing the label content, each printing field is printed on a different adhesive label, said adhesive labels being individually detachable from the common support strip.

This printing mode is particularly advantageous when the labels must be individually put on a plurality of different containers, for example syringes.

As an alternative, not being part of the present invention, in said step of printing the label content, all the printing fields can be printed one after the other on a single adhesive label.

This printing mode allows a single label to be put on a drug bottle containing all the drugs of a specific therapy to be prepared.

In both cases, the labels on which at least one drug printing field is printed are put on containers for the administration of the respective drug to the patient.

The method can advantageously comprise a step of recording the occurred administration of the drug by means of a remote device, that is able to read a graphic identifier of the drug printing field.

This step is particularly advantageous since it suggests a check of the actual administration, allowing reliable statistics on drug consumption to be generated, detecting waste and potential abusive behaviours.

Preferably, the above remote device is located near a hospital bed or otherwise dedicated to a given patient. The device can be for example a tablet attached in close proximity to the bed, or a display screen of any kind located inside the operating room.

Said remote device can thus advantageously notify the operator when the read graphic identifier does not match with a drug comprised in the drug list of the respective patient, warding off possible cases of mistaken identity having harmful outcomes.

Said step of associating a drug list with the patient can advantageously provide a possible sub-step of recalling a drug list previously used for the same patient, with the possibility to modify said drug list by the operator.

Said step of associating a drug list with the patient can also or otherwise provide a possible sub-step of importing the drug list from a preset protocol file, with the possibility to modify said drug list by the operator.

The two above-discussed possibilities advantageously allow the times for generating the drug list by the operator to be reduced.

Preferably the method further comprises a preliminary step of recognizing the operator by the labelling system, that is necessary to enable the following steps of associating a drug list with the patient and/or of printing the generated label content.

Said preliminary step of recognizing can advantageously occur through optical reading of a graphic code printed in advance by the same system after a first identification of the operator through secret credentials.

The operator must thus enter his secret credentials only a first time, and then he can print an identifying label that will contain the graphic code (preferably a barcode, for example a bidimensional barcode) identifying him. The next logins to the system fastly occur by scanning the graphic code through an optical reader associated with the system.

It must be noted that printing a graphic code for identifying the user to log in the printing system itself is an innovative aspect of the system not depending on label printing modes.

A possible divisional application of the present application can therefore be directed to a labelling method comprising the steps of:
arranging a labelling system comprising at least one data input unit, one data processing unit and one printing device;
recognizing a qualified operator;
generating a label content;
printing, through the printing device of said labelling system, the label content generated on one or more adhesive labels located on a support strip;
where the label content comprises a graphic code identifying the operator, preferably a barcode;
where the optical reading of said graphic identifying code allows the operator to be recognized by the system itself and/or by another system requiring the identification of the operator (for example: another limited-access computer system that is present in a hospital setting); and
where, at least a first time, the labelling system identifies the operator through secret credentials or however alternatively to the optical reading of said graphic identifying code.

The step of recognizing the qualified operator thus occurs through optical reading of a graphic code printed in advance by the labelling system after a first identification of the operator through secret credentials.

In this way, it is possible to use the labelling system to generate a label that allows the operator to be fastly identified, both by the system itself and by other limited-access systems. The label can be put on a personal accessory (for example: plate or bracelet) that allows the operator to fastly identify himself if necessary.

In an embodiment of the invention, the step of printing the label content is allowed, at least for some drugs, only if two different qualified operators approve the drug list in advance.

The drug printing fields comprise at least one colour-painted background identifying the type of drug. Yet preferably, the drug printing fields further comprise a graphic code for colour-blind persons that identifies the colour of said painted background.

The above-defined technical problem is further solved by a labelling system for identifying drugs to be administered to a patient in a hospital and/or surgical setting, as defined by the appended claim 12.

The above system is arranged to implement the method of claim 1.

Further features and advantages will become more apparent from the following detailed description of a preferred, but not exclusive, embodiment of the present invention, with reference to the attached figures given by way of non-limiting example.

### Brief description of the drawings

Figure 1 schematically depicts two strips of distinct labels generated by the method and by the system according to the present invention;
Figure 2 schematically depicts two strips of a single label generated by a method according to an example not being part of the present invention;
Figure 3 depicts in detail a header adhesive label printed by the method and by the system according to the present invention;
Figure 4 depicts in detail a drug adhesive label printed by the method and by the system according to the present invention;
Figure 5 schematically depicts the system according to the present invention;
Figure 6 depicts a block diagram of a first operating mode of the method according to the present invention;
Figure 7 depicts a block diagram of a second operating mode of the method according to the present invention;
Figure 8 depicts a block diagram of a third operating mode of the method according to the present invention.

### Detailed description

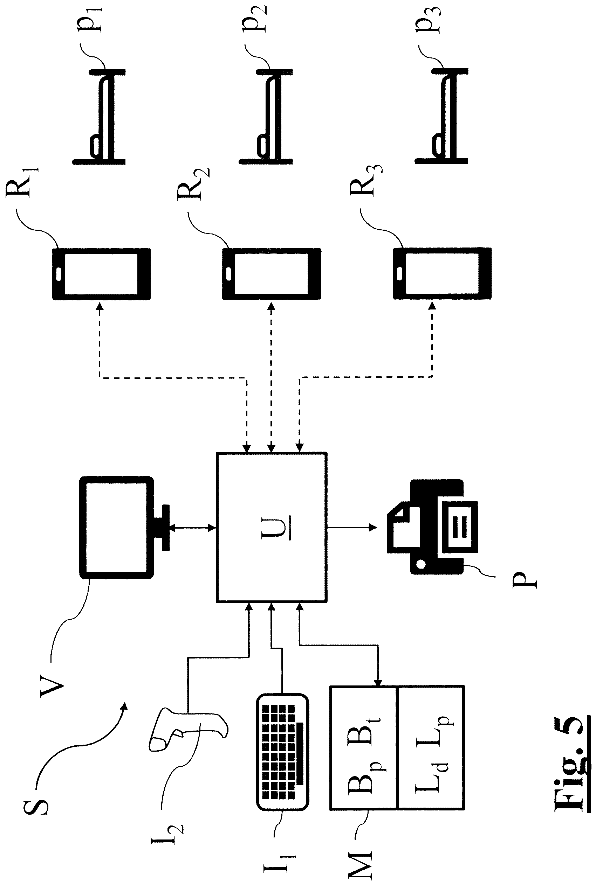

With reference to the attached figure 5, a labelling system for identifying drugs to be administered to a patient in a hospital or surgical setting is generally identified with S.

As stated beforehand in the above introduction, said system can be advantageously employed in several hospital contexts, where the labelling of intermediate containers (for example: syringes, syringe pumps, infusion bags, bottles, and the like) for the administration of drugs of any nature to a patient is to be arranged.

The system comprises at least one data processing unit U, connected to a plurality of input/output peripheral units.

The data processing unit U is arranged to execute a computer program that allows the automated operations of the method according to the present invention to be performed.

It must be noted that the program can be partially or totally executed on one or more remote units that interface with the rest of the system S comprising the peripheral units. These remote units can be general-purpose devices not specifically directed to the aim, for example smartphones on which a specific application was installed.

The peripheral units comprise at least one printing device P, arranged in particular to print adhesive labels 1; 1ₕ; 1_{d}.

The peripheral units further comprise a plurality of data input units I₁; I₂; V that, as it will better come out below in the present description, allow in particular to the operator all those operations allowing a printing content to be generated.

The data input units can comprise a keyboard I₁ and an optical reader I₂. Naturally, other data input units of a known type, such as for example a mouse, can be used as an alternative to or in combination with the previous ones.

A display screen V, whose primary function is to provide a visual support to the operations for generating the printing content, can be of the touch-sensitive type defining an input interface as well.

Moreover, one or more memories M, of the fixed or volatile type, are connected to the data processing unit U. It must be observed that these memory units M can also be remotely located with respect to the data processing unit U. Preferably, said memories are at least partially located on a cloud that can be accessed by the different processing units U interfacing the system S.

The labelling system S can further provide a plurality of fixed remote devices Rᵢ, for example tablets, that are able to exchange data with the data processing unit U. Preferably, for some reasons that will become more apparent below, said remote devices Rᵢ are located in a non-removable manner in close proximity to as many beds. In this way, a single remote device Rᵢ is univocally associated with a recovering patient Pᵢ in the neighbouring bed.

The above-described labelling system S is arranged to generate, upon indication by one or more designated operators, a label content that is printed in colours by the printing device S on an appropriate strip support.

Each label content is directed to a specific patient pᵢ and it describes a drug list tᵢ approved for said patient pᵢ. The drug list tᵢ comprises one or more drugs to be administered to the patient pᵢ: these drugs can define a therapy identified by the designated practitioner or a series of drugs to be taken in an intraoperative setting, for example in the context of a surgical anaesthesia.

The label content comprises a plurality of successive printing fields fₕ, f_{d} that are printed on a strip of label/ s.

A first printing field fₕ, preferably the first of the strip of label/s, is a header field that identifies the patient that is the recipient of the drug list tᵢ.

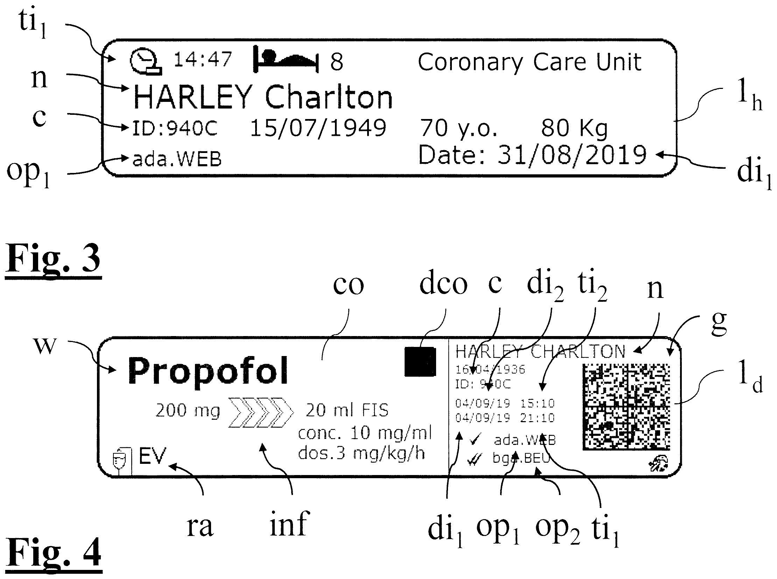

The header printing field fₕ, an example of which is individually represented in figure 3, preferably comprises the patient name n accompanied by his nosocomial code c.

The field can further comprise the printing date di₁ and time tii and an identifying code of the operator op₁ in charge of this printing, in order to facilitate an immediate tracking.

The header printing field fₕ can also comprise other information being useful to identify the patient, for example the recovery ward, bed number, date of birth, age, weight.

The printing fields f_{d} after the first one are instead drug printing fields; each of them concerns a single drug dᵢ of the drug list tᵢ assigned to the patient.

The drug printing field fa, an example of which is individually represented in figure 4, comprises a verbal identifier w of the drug (preferably: the general drug name) and a graphic identifier g thereof.

The latter is preferably a barcode, for example a dimensional barcode. This barcode can contain information of various type about the drug, and it allows in particular the drug to be identified by means of optical reading devices.

In a specific embodiment of the label, the graphic identifier g or the barcode is arranged to be read by a machinery (for example: a syringe pump) arranged to interact with the container which the label will be put on.

The drug printing field f_{d} has a colour-painted background co, that chromatically identifies the type of drug according to the A.S.T.M. D4774-94 international identification code and the DIVI extension thereof. Preferably, in addition to the colour-painted background co a graphic code dco is provided to make the same information about the type of drug immediately available to colour-blind users.

The drug printing field f_{d} can also advantageously indicate different information inf being useful to lead the operator in the preparation of the drug itself, for example: posology, dilution liquid, concentration and dosage.

A graphic and/or verbal identifier of the route of administration of the drug ra can be further provided.

Other information indicated on the drug printing field f_{d} can be once again the printing date di₁ and time tii and an identifying code of the operator opi in charge of this printing.

At least for given drugs, the system S can provide the need of approval by a second authorized operator in addition to the one performing printing. In this case, the drug printing field f_{d} can comprise the date di₂ and time ti₂ of this approval, as well as an ID of the second designated operator op₂.

Moreover, the name n and the nosocomial code c of the patient can be also indicated on the drug printing field f_{d}, - preferably in a smaller typeface.

The label content comprising the different printing fields fₕ, f_{d} can be printed on two printing media of a different type, as needed.

Both printing media comprise an endless support strip 2, preferably unwound from a roll.

In the invention, said support strip 2 has a plurality of separate labels 1ₕ; 1_{d}, equally spaced from each other, that can be individually detached from the strip itself. In an example not being part of the invention, the support strip 2 is instead overhung by an endless adhesive label 1.

According to the type of media being used, label strips of different types are obtained.

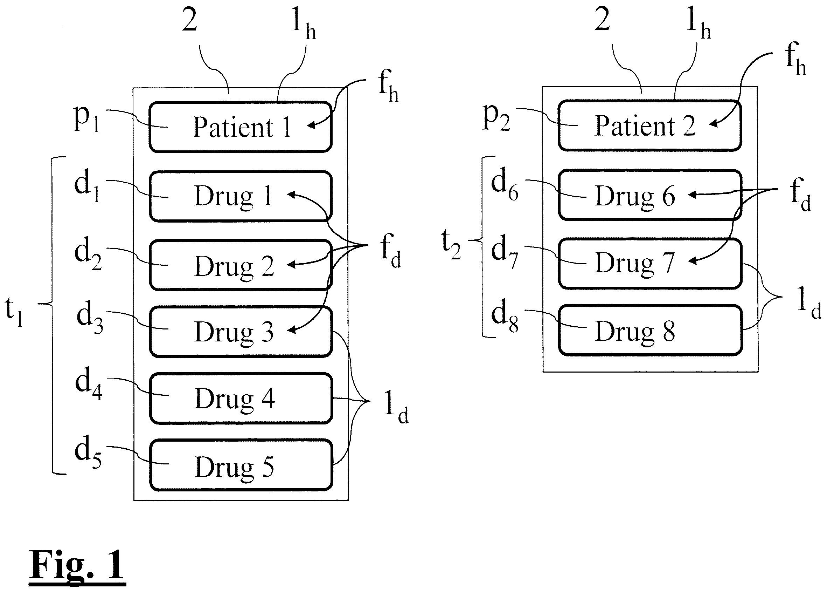

In the invention, illustrated by the example strips of figure 1, the different printing fields fₕ, f_{d} are printed on separate adhesive labels 1ₕ; 1_{d}. The header label 1ₕ indicates the header printing field fₕ, and it only serves to identify the rest of the strip. The following drug labels 1_{d} indicate the drug fields f_{d}, and they must be put on the different containers into which the operator will introduce the drugs dᵢ of the drug list tᵢ.

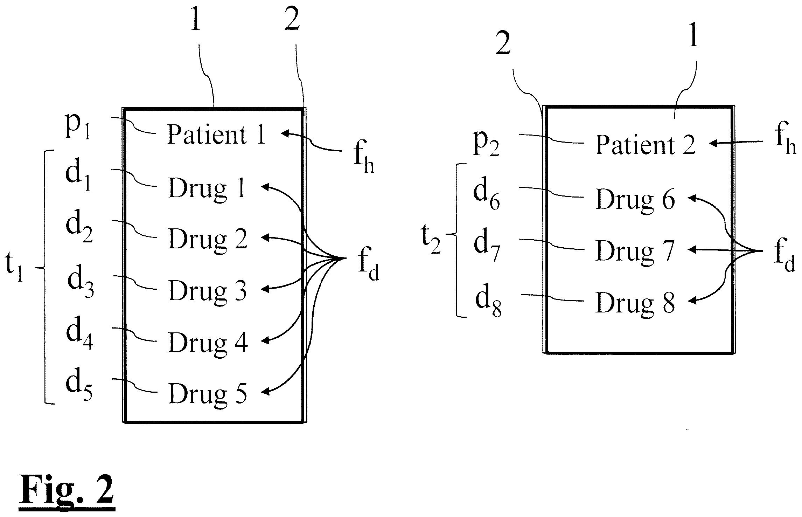

In the example not being part of the invention, illustrated by the example strips of figure 2, the different printing fields fₕ, f_{d} fall in a single label 1, to be put on a drug capsule housing all the drugs dᵢ of the drug list tᵢ.

It is observed that the printing device P provides automated means for cutting the support strip 2: the strip is thus size-cut upon reaching the length required to contain the different printing fields fₕ, f_{d}. In this way, it is avoided that successive strips intended for different patients pᵢ are adjacent, which would generate potential errors.

In the examples of figure 1 and 2, it is observed how the number of fields affects the final length of the support strip 2.

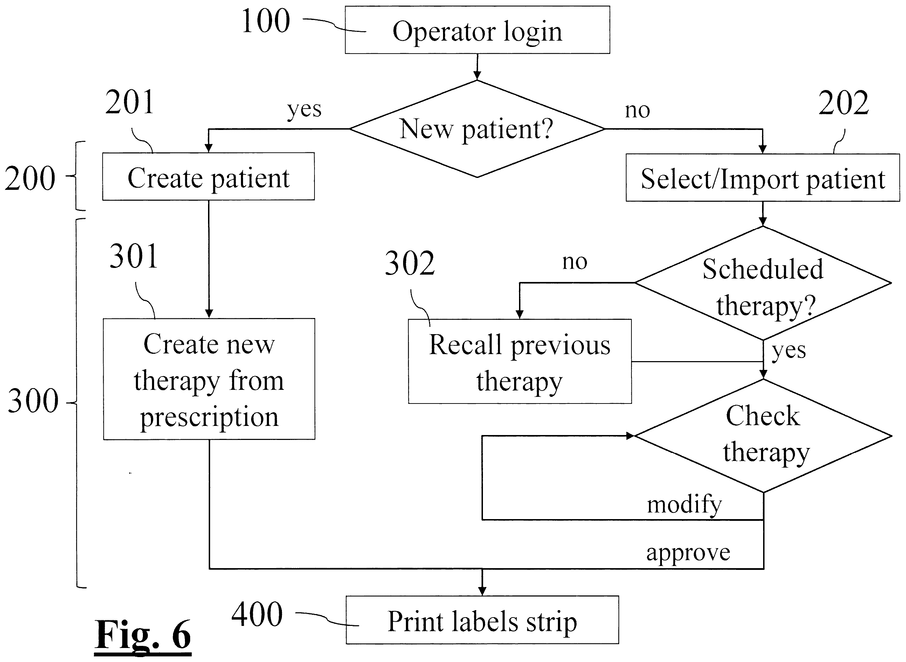

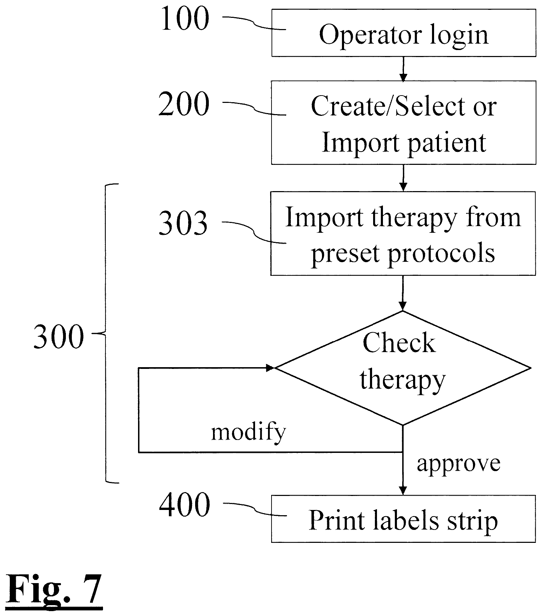

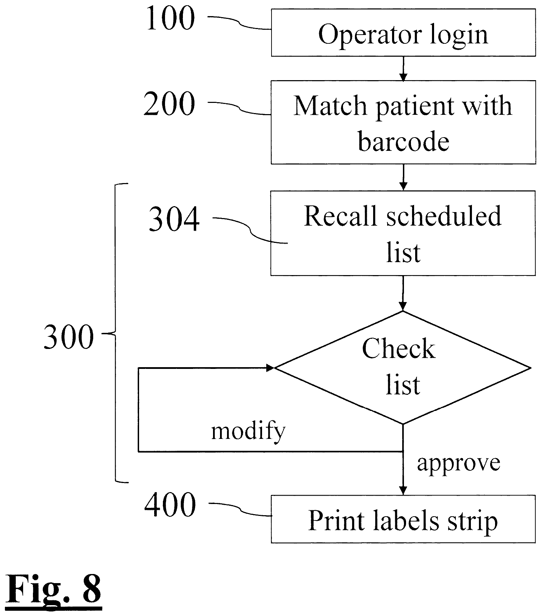

With reference to the attached figures 6-8, the method through which the system S generates and prints the label strips will now be analysed.

A preliminary step 100 provides for the identification of the operator that interfaces with the system S by requiring the printing.

The identification can traditionally occur by entering identifying credentials through the keyboard I₁.

Once a first recognition by the system S is performed, an auxiliary step of the method can provide for the printing, through the printing device P, of a recognition label for the operator. The recognition label comprises a graphic code, preferably a barcode that is recognizable by the optical reader I₂, to log in the system S without manually entering the above credentials. The operator can thus stick the label to a personal accessory, for example a plate or a bracelet, through which he performs the fast identification for the next logins to the system S.

A following step 200 of the method concerns the identification of the patient.

If the patient is a new patient, an appropriate record must be created (step 201) on a patient database Bₚ that is present in the memory M. The patient can be fastly identified also by inserting only the nosocomial code c, that can be read by passing the optical reader I₂ on an identification code indicated for example on a bracelet put on the wrist of the patient.

If on the contrary the patient record has already been created, it must be selected or imported (step 202) by the operator to pursue with the definition of the drug list tᵢ.

The following step provides for the association 300 of a drug list tᵢ with the patient pᵢ.

To do this, the operator has naturally the possibility to create *from scratch* this drug list tᵢ (step 301). This can be advantageously done by displaying on the display screen V the drugs stored in a drug file L_{d}, and selecting them for example by means of a drag and drop interface by using the touch-sensitive screen of the display screen V itself.

As an alternative, the operator can retrieve a therapy (step 302) from a database of the previous drug lists Bt, that contains the therapies previously administered for which the system has printed the related labels. Preferably, in this case the system S automatically recalls the drug lists tᵢ previously intended for the same patient pᵢ. The operator can then decide to keep or modify the drug list tᵢ before the final approval.

The operator can also recall a protocol (step 303) from a protocol file Lₚ, that comprises therapeutic protocols used in the concerned ward or drug lists applied during given surgeries. In this case too, the drug list tᵢ from the protocol can be modified in case before the approval.

It must be noted that the entry and/or approval of a drug list do not involve an immediate printing. When a surgery on the patient is planned, for example, the drug list tᵢ can be entered and/or approved before the actual surgery. In this case, in a next login to the system the entered and/or approved drug list is recalled (step 304) to proceed then to the printing.

It must be noted that the operators qualified to approve a drug list can belong to a narrower group or anyhow different from the one of the operators qualified to print. Moreover, as hinted above, for some or all the types of drug a double approval can be required before the final printing.

A step 400 following the above-described ones is printing the label content comprising the approved drug list tᵢ, that generates a strip of adhesive labels 1; 1ₕ; 1_{d} of the above-described type. These labels must thus be suitably stuck on the related containers.

At the time of printing a label content comprising an approved drug list tᵢ, this list is stored by the system S, so as to keep track of the administered drugs.

Moreover, after printing, a display of the drug list tᵢ can be sent to a local device in close proximity to the place where the drugs must be administered. In the case of a recovering patient pᵢ, the drug list tᵢ is sent to the remote device Rᵢ near the bed assigned to the patient pᵢ himself; in the case of a patient pᵢ waiting for surgery, the drug list tᵢ is sent to a display screen located in the operating room.

The possibility to check the actual administration of the drugs dᵢ to the patient can be provided. The check occurs by means of the operator by interfacing the above local device. The check can advantageously occur through optical scanning of the graphic identifier g of the drug dᵢ printed on the drug adhesive label 1_{d}.

Advantageously, in these cases the system can also carry out a consistency check about the drug list tᵢ approved for the patient pᵢ, so as to notify the operator if the drug dᵢ to be administered is dedicated to another patient pᵢ.

The above-described system S allows a telematic register to be kept constantly updated with all the operations performed by the operators, concerning in particular the printing of drugs lists tᵢ for related patients pᵢ. Starting from these data, it is possible to carry out statistics on the use of drugs, detecting waste and possible thefts.

The above-described system S can further allow miscellaneous labels to be printed, that are different from the above-hinted ones, customizable with date, time, operators, patient name and record data. Moreover, it is possible to create miscellaneous labels to label containers or the like, for example sterilization containers or milk bottles, or drugs, or even customized labels of a different kind. The printing of these custom labels can be enriched with the printing date and time and the operator reference; however, the printing operation is still stored in the telematic register of the system S.

The above-described system S can also provide a module for filling and managing the patient data and therapy.

The patient data and therapy (the so-called therapy sheets), filled in by a qualified operator, contain the treatment plan for each patient with the indication of the drugs to be taken at the different times of the day. Starting from said therapy sheet, the system S can automatically generate a drug list tᵢ to be printed comprising the drugs to be taken in a given time-slot that require a labelling.

In this case, the operator that logs in the labelling system S recalls this drug list tᵢ and proceeds to the printing.

Advantageously, the terminal arranged to fill in the therapy sheets can be unique, for example located in an office dedicated to the medial staff, while the printing terminals can be deployed in the hospital and also accessed by the other healthcare operators.

The labelling system S can also interface with another system for filling in medical records, for example a management software of the known type, automatically importing the data of the medical records required for the generation of the drug lists tᵢ for the various patients pᵢ.

The labelling system S can also comprise appropriate functions for managing a local drug warehouse of the ward.

In this case, the system stores an inventory of the drug packages that are present in the local warehouse: each field comprises the expiry date and the residual package content.

Moreover, the system S generates a label to be put on each package. The label comprises at least one recognition code that is readable through optical scanning (for example: a bidimensional barcode).

When an operator collects one or more drugs from the warehouse, he uses the system optical reader I₂ to scan the label on the package, pointing out the number of collected doses. The system S then automatically updates the inventory.

Moreover, the system S can notify the user in case the same drug is available in a package having an earlier expiry date.

The system can further display and/or print a summary report of the drugs that are present in the local warehouse with the related expiry dates.

Obviously, in order to meet contingent and specific requirements, a person skilled in the art will be allowed to bring various modifications and alternatives to the above-described invention.

## Claims

1. Labelling method for identifying drugs to be administered to a patient in a hospital or surgical setting, comprising the steps of:
arranging a labelling system (S) comprising at least one data input unit (I₁; I₂; V), one data processing unit (U) and one printing device (P);
identifying (200) a patient (pᵢ) through the data input unit (I₁; I₂; I₃);
associating (300) a drug list (tᵢ) with said patient, said drug list (tᵢ) comprising a set of drugs (dᵢ) to be administered to the patient;
generating, through the data processing unit (U), a label content comprising at least one printing field (f_{d}) for each drug (dᵢ) of the drug list (tᵢ), each of said printing fields (f_{d}) comprising at least one identifier of the respective drug (dᵢ);
printing (400), through the printing device (P) of said labelling system (S),
the generated label content on one or more adhesive labels (1ₕ; 1_{d}) located on a common support strip (2), so that the identifiers (w; g) of all the drugs (dᵢ) of the drug list (tᵢ) are indicated on said support strip (2); each printing field (fₕ, f_{d}) being printed on a different adhesive label (1ₕ, 1_{d}), said adhesive labels (1ₕ, 1_{d}) being individually detachable from the common support strip (2), **characterized in that**:
said support strip (2) is unwound from an endless feeding strip, said method further comprising a step of automatically size-cutting said endless feeding strip which ensures that the strips related to different patients are separated from each other; and
the drug printing fields (f_{d}) comprise at least one colour-painted background (co) identifying the type of drug (f_{d}).

2. Labelling method according to any of the preceding claims, wherein the generated and printed printing content further comprises a header printing field (fₕ), comprising the name (n) and/or an ID (c) of the patient (Pi).

3. Labelling method according to any of the preceding claims, further comprising a step of putting the labels (1ₕ; 1_{d}) on which at least one drug printing field (f_{d}) is printed on containers for the administration of the respective drug to the patient.

4. Labelling method according to claim 3, further comprising a step of recording the occurred administration of the drug by means of a remote device (Rᵢ), that is able to read a graphic identifier (g) of the drug printing field (f_{d}).

5. Labelling method according to claim 4, wherein said remote device (Rᵢ) is located near a hospital bed or otherwise dedicated to a given patient (pᵢ), said remote device (Rᵢ) notifying an operator when the read graphic identifier (g) does not match with a drug (dᵢ) comprised in the drug list (tᵢ) of the respective patient (pᵢ).

6. Labelling method according to any of the preceding claims, wherein said step of associating (300) a drug list (tᵢ) with the patient (pᵢ) provides a possible sub-step of recalling (302) a drug list previously used for the same patient (pᵢ), with the possibility to modify said drug list by an operator.

7. Labelling method according to any of the preceding claims, wherein said step of associating (300) a drug list (tᵢ) with the patient (pᵢ) provides a possible sub-step of importing (303) the drug list from a memory (Lₚ) of preset protocols, with the possibility to modify said drug list (tᵢ) by an operator.

8. Labelling method according to any of the preceding claims, further comprising a preliminary step of recognizing (100) an operator by the labelling system (S), that is necessary to enable the following steps of associating (300) a drug list (tᵢ) with the patient (pᵢ) and/or of printing (400) the generated label content.

9. Labelling method according to claim 8, wherein said preliminary step of recognizing (100) occurs through optical reading of a graphic code printed in advance by the same system (S) after a first identification of the operator through secret credentials.

10. Labelling method according to any of claims 8 or 9, wherein the step of printing (400) the label content is allowed only if two different qualified operators previously approve the drug list (tᵢ).

11. Labelling method according to any of the preceding claims, wherein the drug printing fields (f_{d}) comprise one graphic code for colour-blind persons (dco) that identifies the colour of said painted background (co).

12. Labelling system (S) for identifying drugs to be administered to a patient in a hospital and/or surgical setting, comprising at least:
one data input unit (I₁; I₂; V) arranged at least to allow a patient (pᵢ) to be identified;
one data processing unit (U) configured to generate a label content comprising at least one printing field (f_{d}) for each drug (dᵢ) of a drug list (tᵢ) to be administered to the patient (pᵢ), each of said printing fields (f_{d}) comprising at least one identifier of the respective drug (dᵢ);
one printing device (P) configured to print the label content on one or more adhesive labels (1ₕ; 1_{d}) located on a common support strip (2), so that the identifiers (w; g) of all the drugs (dᵢ) of the drug list (tᵢ) are indicated on said support strip (2) each printing field (fₕ, f_{d}) being printed on a different adhesive label (1ₕ, 1_{d}), said adhesive labels (1ₕ, 1_{d}) being individually detachable from the common support strip (2); the labelling system **characterized in that**:
said support strip (2) is unwound from an endless feeding strip, and **in that** the labelling system comprises automated means for cutting the support strip (2) which ensure that the strips related to different patients are separated from each other; and
wherein the drug printing fields (f_{d}) comprise: at least one colour-painted background (co) identifying the type of drug (f_{d}).

## Patentansprüche

1. Etikettierverfahren zum Identifizieren von an einen Patienten in einem Krankenhaus oder einer chirurgischen Umgebung zu verabreichenden Arzneimitteln, das die Schritte aufweist von:
Anordnen eines Etikettiersystems (S), das mindestens eine Dateneingabeeinheit (I₁; I₂; V), eine Datenverarbeitungseinheit (U) und eine Druckvorrichtung (P) aufweist;
Identifizieren (200) eines Patienten (pᵢ) durch die Dateneingabeeinheit (I₁; I₂; I₃);
Zuordnen (300) einer Arzneimittelliste (tᵢ) zu dem Patienten, wobei die Arzneimittelliste (tᵢ) einen Satz von Arzneimitteln (dᵢ) aufweist, die dem Patienten zu verabreichen sind;
Erzeugen, durch die Datenverarbeitungseinheit (U), eines Etiketteninhalts, der mindestens ein Druckfeld (f_{d}) für jedes Arzneimittel (dᵢ) der Arzneimittelliste (tᵢ) aufweist, wobei jedes der Druckfelder (f_{d}) mindestens eine Kennung des jeweiligen Arzneimittels (dᵢ) aufweist;
Drucken (400), durch die Druckvorrichtung (P) des Etikettiersystems (S), des erzeugten Etiketteninhalts auf eines oder mehrere Klebeetiketten (1ₕ; 1_{d}), die sich auf einem gemeinsamen Trägerstreifen (2) befinden, sodass die Kennungen (w; g) aller Arzneimittel (dᵢ) der Arzneimittelliste (tᵢ) auf dem Trägerstreifen (2) angegeben werden; wobei jedes Druckfeld (fₕ, f_{d}) auf ein anderes Klebeetikett (1ₕ, 1_{d}) gedruckt wird, wobei die Klebeetiketten (1ₕ, 1_{d}) einzeln von dem gemeinsamen Trägerstreifen (2) ablösbar sind, **dadurch gekennzeichnet, dass**:
der Trägerstreifen (2) von einem Endloszuführstreifen abgerollt wird, wobei das Verfahren ferner einen Schritt des automatischen Schneidens des Endloszuführstreifens aufweist, der sicherstellt, dass die Streifen, die verschiedenen Patienten zugeordnet sind, voneinander getrennt werden; und
die Arzneimitteldruckfelder (f_{d}) mindestens einen farbigen Hintergrund (co) aufweisen, der die Art des Arzneimittels (f_{d}) identifiziert.

2. Etikettierverfahren nach einem der vorhergehenden Ansprüche, wobei der erzeugte und gedruckte Druckinhalt ferner ein Kopfdruckfeld (fₕ) aufweist, das den Namen (n) und/oder eine ID (c) des Patienten (pᵢ) enthält.

3. Etikettierverfahren nach einem der vorhergehenden Ansprüche, das ferner einen Schritt des Anbringens der Etiketten (1ₕ; 1_{d}), auf denen mindestens ein Arzneimitteldruckfeld (f_{d}) aufgedruckt ist, an Behältern für die Verabreichung des jeweiligen Arzneimittels an den Patienten aufweist.

4. Etikettierverfahren nach Anspruch 3, das ferner einen Schritt des Aufzeichnens der erfolgten Verabreichung des Arzneimittels mittels einer Fern-Vorrichtung (Rᵢ) aufweist, die in der Lage ist, eine grafische Kennung (g) des Arzneimitteldruckfeldes (f_{d}) zu lesen.

5. Etikettierverfahren nach Anspruch 4, wobei sich die Fern-Vorrichtung (Rᵢ) in der Nähe eines Krankenhausbettes befindet oder auf andere Weise einem bestimmten Patienten (pᵢ) zugeordnet ist, wobei die Fern-Vorrichtung (Rᵢ) eine Bedienungsperson benachrichtigt, wenn die gelesene grafische Kennung (g) nicht mit einem Arzneimittel (dᵢ) übereinstimmt, das in der Arzneimittelliste (tᵢ) des jeweiligen Patienten (pᵢ) enthalten ist.

6. Etikettierverfahren nach einem der vorhergehenden Ansprüche, wobei der Schritt des Zuordnens (300) einer Arzneimittelliste (tᵢ) zu dem Patienten (pᵢ) einen möglichen Teilschritt des Abrufens (302) einer zuvor für denselben Patienten (pᵢ) verwendeten Arzneimittelliste mit der Möglichkeit vorsieht, die Arzneimittelliste durch eine Bedienungsperson zu ändern.

7. Etikettierverfahren nach einem der vorhergehenden Ansprüche, wobei der Schritt des Zuordnens (300) einer Arzneimittelliste (tᵢ) zu dem Patienten (pᵢ) einen möglichen Teilschritt des Importierens (303) der Arzneimittelliste aus einem Speicher (Lₚ) von voreingestellten Protokollen mit der Möglichkeit vorsieht, die Arzneimittelliste (tᵢ) durch eine Bedienungsperson zu ändern.

8. Etikettierverfahren nach einem der vorhergehenden Ansprüche, das ferner einen vorbereitenden Schritt des Erkennens (100) einer Bedienungsperson durch das Etikettiersystem (S) aufweist, der notwendig ist, um die folgenden Schritte des Zuordnens (300) einer Arzneimittelliste (tᵢ) zu dem Patienten (pᵢ) und/oder des Druckens (400) des erzeugten Etiketteninhalts zu ermöglichen.

9. Etikettierverfahren nach Anspruch 8, wobei der vorbereitende Schritt des Erkennens (100) durch optisches Lesen eines grafischen Codes erfolgt, der im Voraus von dem gleichen System (S) nach einer ersten Identifizierung der Bedienungsperson durch geheime Zugangsdaten gedruckt wird.

10. Etikettierverfahren nach einem der Ansprüche 8 oder 9, wobei der Schritt des Druckens (400) des Etiketteninhalts nur dann zulässig ist, wenn zwei verschiedene qualifizierte Bedienungspersonen die Arzneimittelliste (tᵢ) zuvor genehmigen.

11. Etikettierverfahren nach einem der vorhergehenden Ansprüche, wobei die Arzneimitteldruckfelder (f_{d}) einen grafischen Code für Farbenblinde (dco) enthalten, der die Farbe des farbigen Hintergrunds (co) identifiziert.

12. Etikettiersystem (S) zum Identifizieren von an einen Patienten in einem Krankenhaus und/oder einer chirurgischen Umgebung zu verabreichenden Arzneimitteln, das mindestens aufweist:
eine Dateneingabeeinheit (I₁; I₂; V), die dazu eingerichtet ist, zumindest die Identifizierung eines Patienten (pᵢ) zu ermöglichen;
eine Datenverarbeitungseinheit (U), die dazu ausgelegt ist, einen Etiketteninhalt zu erzeugen, der mindestens ein Druckfeld (f_{d}) für jedes Arzneimittel (dᵢ) einer Arzneimittelliste (tᵢ) aufweist, das dem Patienten (pᵢ) zu verabreichen ist, wobei jedes der Druckfelder (f_{d}) mindestens eine Kennung des jeweiligen Arzneimittels (dᵢ) aufweist;
eine Druckvorrichtung (P), die dazu ausgelegt ist, den Etiketteninhalt auf eines oder mehrere Klebeetiketten (1ₕ; 1_{d}) zu drucken, die sich auf einem gemeinsamen Trägerstreifen (2) befinden, sodass die Kennungen (w; g) aller Arzneimittel (dᵢ) der Arzneimittelliste (tᵢ) auf dem Trägerstreifen (2) angegeben werden, wobei jedes Druckfeld (fₕ, f_{d}) auf ein anderes Klebeetikett (1ₕ, 1_{d}) gedruckt wird, wobei die Klebeetiketten (1ₕ, 1_{d}) einzeln von dem gemeinsamen Trägerstreifen (2) ablösbar sind;
wobei das Etikettiersystem **dadurch gekennzeichnet ist, dass**:
der Trägerstreifen (2) von einem Endloszuführstreifen abgerollt wird und das Etikettiersystem eine automatisierte Einrichtung zum Schneiden des Trägerstreifens (2) aufweist, die sicherstellt, dass die Streifen, die verschiedenen Patienten zugeordnet sind, voneinander getrennt werden; und
wobei die Arzneimitteldruckfelder (f_{d}) mindestens einen farbigen Hintergrund (co) aufweisen, der die Art des Arzneimittels (f_{d}) identifiziert.

## Revendications

1. Procédé d'étiquetage pour l'identification des médicaments à administrer à un patient en milieu hospitalier ou chirurgical, comprenant les étapes suivantes :
mise en place d'un système d'étiquetage (S) comprenant au moins une unité d'entrée de données (I₁ ; I₂; η, une unité de traitement des données (U) et un dispositif d'impression (P) ;
identifier (200) un patient (pᵢ) par l'intermédiaire de l'unité d'entrée des données (I₁ ; I₂; I₃) ;
associer (300) une liste de médicaments (tᵢ) audit patient, ladite liste de médicaments (tᵢ) comprenant un ensemble de médicaments (dᵢ) à administrer au patient ;
générer, par l'intermédiaire de l'unité de traitement de données (U), un contenu d'étiquette comprenant au moins un champ d'impression (f_{d}) pour chaque médicament (dᵢ) de la liste de médicaments (tᵢ),
chacun desdits champs d'impression (f_{d}) comprenant au moins un identificateur du médicament respectif (dᵢ) ;
impression (400), par l'intermédiaire du dispositif d'impression (P) dudit système d'étiquetage (S), le contenu d'étiquette généré sur une ou plusieurs étiquettes adhésives (1ₕ ; 1_{d}) prévue sur une bande support (2) commune, de manière à ce que les identificateurs (w ; g) de tous les médicaments (dᵢ) de la liste des médicaments (tᵢ) soient indiqués sur ladite bande support (2) ; chaque champ d'impression (fₕ, f_{d}) étant imprimé sur une étiquette adhésive (1ₕ, 1_{d}) différente, lesdites étiquettes adhésives (1ₕ, 1_{d}) étant individuellement détachables de la bande support commune (2), **caractérisé en ce que** :
ladite bande support (2) est déroulée à partir d'une bande d'alimentation sans fin, ledit procédé comprenant en outre une étape de découpage automatique de la taille de ladite bande d'alimentation sans fin qui garantit que les bandes relatives à différents patients sont séparées les unes des autres ; et
les champs d'impression (f_{d}) des médicaments comprennent au moins un arrière-plan coloré (co) identifiant le type de médicament (f_{d}).

2. Procédé d'étiquetage selon l'une quelconque des revendications précédentes, selon lequel le contenu d'impression généré et imprimé comprend en outre un champ d'impression d'en-tête (fₕ), comprenant le nom (n) et/ou un ID (c) du patient (pᵢ).

3. Procédé d'étiquetage selon l'une quelconque des revendications précédentes, comprenant en outre une étape de placement des étiquettes (1h ; 1d) sur lesquelles au moins un champ d'impression (f_{d}) de médicament est imprimé sur des récipients pour l'administration du médicament respectif au patient.

4. Procédé d'étiquetage selon la revendication 3, comprenant en outre une étape d'enregistrement de l'administration du médicament au moyen d'un dispositif à distance (Rᵢ) capable de lire un identificateur graphique (g) du champ d'impression (f_{d}) du médicament.

5. Procédé d'étiquetage selon la revendication 4, selon lequel ledit dispositif à distance (Rᵢ) est situé à proximité d'un lit d'hôpital ou dédié d'une autre manière à un patient donné (pᵢ), ledit dispositif à distance (Rᵢ) notifiant un opérateur lorsque l'identifiant graphique lu (g) ne correspond pas à un médicament (dᵢ) figurant dans la liste des médicaments (tᵢ) du patient respectif (pᵢ).

6. Procédé d'étiquetage selon l'une quelconque des revendications précédentes, selon lequel l'étape d'association (300) d'une liste de médicaments au patient (pᵢ) prévoit une éventuelle sous-étape de rappel (302) d'une liste de médicaments précédemment utilisée pour le même patient (pᵢ), avec la possibilité de modifier ladite liste de médicaments par un opérateur.

7. Procédé d'étiquetage selon l'une quelconque des revendications précédentes, selon lequel l'étape d'association (300) d'une liste de médicaments (tᵢ) au patient (pᵢ) prévoit une éventuelle sous-étape d'importation (303) de la liste de médicaments à partir d'une mémoire (Lₚ) de protocoles prédéfinis, avec la possibilité de modifier ladite liste de médicaments (tᵢ) par un opérateur.

8. Procédé d'étiquetage selon l'une quelconque des revendications précédentes, comprenant en outre une étape préliminaire de reconnaissance (100) d'un opérateur par le système d'étiquetage (S), nécessaire pour permettre les étapes suivantes d'association (300) d'une liste de médicaments (tᵢ) au patient (pᵢ) et/ou d'impression (400) du contenu de l'étiquette générée.

9. Procédé d'étiquetage selon la revendication 8, selon lequel ladite étape préliminaire de reconnaissance (100) se produit par lecture optique d'un code graphique imprimé à l'avance par le même système (S) après une première identification de l'opérateur au moyen d'informations d'identification secrètes.

10. Procédé d'étiquetage selon l'une des revendications 8 ou 9, selon lequel l'étape d'impression (400) du contenu de l'étiquette n'est autorisée que si deux différents opérateurs qualifiés approuvent au préalable la liste de médicaments (tᵢ).

11. Procédé d'étiquetage selon l'une quelconque des revendications précédentes, dans lequel les champs d'impression (f_{d}) des médicaments comprennent un code graphique pour daltoniens (dco) qui identifie la couleur dudit arrière-plan coloré (co).

12. Système d'étiquetage (S) pour identifier les médicaments à administrer à un patient dans un environnement hospitalier et/ou chirurgical, comprenant au moins :
une unité d'entrée de données (Iᵢ ; I₂ ; η agencée au moins pour permettre l'identification d'un patient (Pi) ;
une unité de traitement de données (U) configurée pour générer un contenu d'étiquette comprenant au moins un champ d'impression (f_{d}) pour chaque médicament (dᵢ) d'une liste de médicaments (tᵢ) à administrer au patient (pi), chacun desdits champs d'impression (f_{d}) comprenant au moins un identificateur du médicament respectif (dᵢ) ;
un dispositif d'impression (P) configuré pour imprimer le contenu de l'étiquette sur une ou plusieurs étiquettes adhésives (1ₕ ; 1_{d}) prévues sur une bande support (2) commune, de manière à ce que les identificateurs (w ; g) de tous les médicaments (dᵢ) de la liste des médicaments (tᵢ) soient indiqués sur ladite bande support (2), chaque champ d'impression (fₕ, f_{d}) étant imprimé sur une étiquette adhésive différente (1ₕ, 1_{d}), lesdites étiquettes adhésives (1ₕ, 1_{d}) étant individuellement détachables de la bande support commune (2),
le système d'étiquetage étant **caractérisé en ce que** :
ladite bande de support (2) est déroulée à partir d'une bande d'alimentation sans fin, et **en ce que** le système d'étiquetage comprend des moyens automatisés de découpe de la bande support (2) qui garantissent que les bandes relatives à différents patients sont séparées les unes des autres ; et dans lequel les champs d'impression des médicaments (f_{d}) comprennent : au moins un arrière-plan coloré (co) identifiant le type de médicament (f_{d}).
